# EUROPÄISCHE PATENTSCHRIFT

(11) **EP 2 645 941 B1**
(45) Veröffentlichungstag und Bekanntmachung des Hinweises auf die Patenterteilung: **08.03.2017**
(21) Anmeldenummer: 11818942.2
(22) Anmeldetag: 30.11.2011
(51) Int. Cl.: A61B 17/04

(54) **VORRICHTUNG ZUR MENISKUSREFIXATION**
DEVICE FOR MENISCUS REATTACHMENT
DISPOSITIF DE REFIXATION DU MÉNISQUE

(30) Priorität: 30.11.2010 DE 102010060899
(43) Veröffentlichungstag der Anmeldung: 09.10.2013
(73) Patentinhaber: Medacta International S.A., 6874 Castel San Pietro (TI) (CH)
(72) Erfinder: LEIBER, Valentin, 78532 Tuttlingen (DE)
(74) Vertreter: Inchingalo, Simona
(86) Internationale Anmeldenummer: PCT/EP2011/005994
(87) Internationale Veröffentlichungsnummer: WO 2012/072244

(56) Entgegenhaltungen:
- WO-A1-00/40159
- WO-A1-97/07743
- WO-A2-03/071962
- US-A1- 2007 142 838

## Beschreibung

### TECHNISCHES GEBIET

Die Erfindung betrifft eine Vorrichtung nach den Merkmalen des Oberbegriffs des Anspruchs 1.

### STAND DER TECHNIK

Aus dem Stand der Technik sind eine Vielzahl von Vorrichtungen und Verfahren zur Meniskusrefixation bekannt. Hierzu werden eine Mehrzahl von Ankern bzw. Fadenankern als Implantate in die Ränder des Risses eingebracht und der Riss mittels eines Fadens refixiert. Zum Einbringen des Ankers wird eine Nadel mit einem Anker und einem Faden bestückt. Die Nadel wird nach hinten in Einstichrichtung aus dem Anker entfernt, der Faden wird an dem Anker verknotet. Dieser Konten verbleibt nach dem Entfernen der Nadeln und dem Abschneiden des Fadens störend und reizend im Gewebe zurück. Der Knoten schleift sich ausserdem in das Knorpelgewebe oder auch in angrenzendes Knochengewebe ein. Bei heutigen Operationstechniken und Verfahren wird das Einschleifen der Knoten und Fadenenden in umliegendes Gewebe toleriert, da keine besseren Verfahren oder Lösungsmöglichkeiten bekannt sind.

Die Merkmale des Oberbegriffs des Anspruchs 1 sind aus WO-A-00/40159 bekannt.

### BESCHREIBUNG DER ERFINDUNG

Aufgabe der Erfindung ist es, eine Vorrichtung zu schaffen, die die o. g. Nachteile behebt oder zumindest minimiert.

Erfindungsgemäss umfasst eine Vorrichtung zur Meniskusrefixation eine Nadel und ein Implantat, wobei das Implantat eine abspreizbare Teilfläche aufweist. Bevorzugt ist das Implantat als Rohrstück ausgebildet, das auf einem Schaft der Nadel aufgenommen ist. Durch die proximalseitige Abspreizung der Teilflächen, in der Fachliteratur auch als Flügel bezeichnet, wird sichergestellt, dass ein unbeabsichtigtes Entfernen oder Herausziehen des Implantats unmöglich wird. Proximal bedeutet hierbei ein Abspreizen in das Knorpelgewebe angrenzend an den Meniskus

In der Fachliteratur wird für das Bauteil, das in der vorliegenden Anmeldung als Implantat bezeichnet wird, häufig auch der Begriff Anker oder Fadenanker gebraucht. In der vorliegenden Anmeldung werden die drei Begriffe parallel verwendet.

Erfindungsgemäss umfasst die Vorrichtung zur Meniskusrefixation einen Spreizring, geeignet die Teilfläche des Implantats abzuspreizen. Dadurch ergibt sich der Vorteil, dass die Teilflächen erst abgespreizt werden können, wenn das Implantat richtig positioniert ist. Ein weiterer Vorteil ist, dass das Implantat mit nicht abgespreizten Teilflächen wesentlich schonender für das umliegende Gewebe positioniert werden kann. Zweckmässigerweise ist der Spreizring als Rohrstück ausgebildet und weist eine Aussenkontur auf, die geeignet ist, die Teilflächen des Implantats abzuspreizen.

Zweckmässigerweise sind sowohl das Implantat, wie auch der Spreizring auf einen Schaft der Nadel angenommen. Bevorzugt weist die Nadel einen Absatz auf, der als Anschlag für das Implantat dient. Über eine Bedieneinrichtung kann der Spreizring in das Implantat eingetrieben werden, so dass er die abspreizbaren Teilflächen des Implantats abspreizt. Besonders bevorzugt handelt es sich um eine konusförmige oder teilkonusförmige Ausbildung des Spreizrings.

Erfindungsgemäss umfasst das Implantat einen Faden und ist der Spreizring geeignet den Faden an dem Implantat zu fixieren. Dadurch ergibt sich der Vorteil, dass mit der Vorrichtung ein knotenfreies Vernähen bzw. eine knotenfreie Meniskusrefixation möglich ist.

Zweckmässigerweise verschliesst der Spreizring das Implantat mit dem Faden so, dass auch das Implantat selbst keine Scheuerstellen für das umliegende Gewebe aufweist. Besonders bevorzugt verbleibt auch der Spreizring mit dem Implantat in dem Gewebe.

Zweckmässigerweise umfasst die Vorrichtung eine Druckstange. Bevorzugt ist die Druckstange geeignet den Spreizring in das Implantat einzubringen. Besonders bevorzugt ist die Druckstange ein Rohrstück, das ebenfalls auf dem Schaft der Nadel aufgenommen ist, und an eine hintere Kante des Spreizrings anschliesst. Dadurch ergibt sich der Vorteil, dass die Vorrichtung sehr einfach aufgebaut ist.

In typischen Ausführungsbeispielen ist die Nadel, insbesondere ein Vorderteil der Nadel durch eine Kraft plastisch verformbar. Bevorzugt wirkt die Kraft auf die Nadel bzw. das Vorderteil der Nadel, wenn der Speizring in das Implantant gepresst wurde und durch die Druckstange weiter das Implantat in Einbringrichtung bewegt. Durch die hervorgerufene plastische Verforumung der Nadel wird das Implantat nach distal über die deformierte Nadel geschoben. Dabei verhaken sich die abspreizbaren Teilflächen im Gewebe. Die Nadel kann anschließend entgegen der Einbringrichtung aus dem Gewebe gezogen werden.

Zweckmässigerweise ist die Nadel dazu aus einem Material gebildet, das ausreichend hart zum Einbringen in das Gewebe, insbesondere Meniskusgewebe, ist.

Zweckmässigerweise umfasst die Vorrichtung einen Rohrschaft, geeignet zur Aufnahme der Nadel. Bevorzugt sind auch das Implantat und der Spreizring und zumindest ein Teil des Druckstücks in dem Rohrschaft aufgenommen. Dadurch ergibt sich der Vorteil, dass die Bauteile in dem Rohrschaft geschützt sind. Des Weiteren ist auch vorteilhaft, dass die Vorrichtung durch den Rohrschaft leichter in das Gelenk eingebracht werden kann.

In typischen Ausführungsbeispielen sind zwei Nadeln, die jeweils mit einem Faden, einem Anker und einem Spreizring bestückt sind, in dem Rohrschaft aufgenommen. Dadurch ergibt sich der Vorteil, dass bei einer Lesion zwei Verankerungspunkte am Meniskus gesetzt werden können.

Bevorzugt sind die beiden Nadeln über eine Fadenbrücke verbunden. Dadurch ergibt sich der Vorteil, dass der Riss im Meniskus durch Anziehen des Fadens repositioniert werden kann. Bevorzugt ist dazu der Faden im zweiten Ankerpunkt bzw. am zweiten Implantat zwischen dem Spreizring und dem Implantat der zweiten Nadel noch nicht festgelegt, sondern kann an diesen noch positioniert und angezogen werden.

Zweckmässigerweise sind die Bauteile oder ist zumindest ein Teil der Bauteile der Vorrichtung aus PEEK (Polyetherketone) oder einem ähnlichen Kunststoff, insbesondere Kunststoffen mit Medizinzulassung hergestellt. Bevorzugt werden Materialien verwendet, die dem "Medical Grade" entsprechen.

In typischen Ausführungsbeispielen ist die Druckstange in einem distalen Bereich flexibel ausgebildet, bevorzugt als Spiraldraht. Besonders bevorzugt umfasst die flexible Druckstange eine starre Druckplatte. Dadurch ergibt sich der Vorteil, dass eine kontrollierte Krafteinleitung auch entlang einer gebogenen Nadel sichergestellt ist.

Gesondert beschrieben wird eine Verwendung einer Vorrichtung mit den beschriebenen Merkmalen zur Meniskusrefixation.

### KURZE BESCHREIBUNG DER ZEICHNUNGEN

Nachfolgend wird die Erfindung anhand der beiliegenden Figuren kurz beschrieben, wobei die Figuren zeigen:
Figur 1 zeigt eine schematische Darstellung einer perspektivischen Explosionsdarstellung einer erfindungsgemässen Vorrichtung;.
Figur 2 zeigt eine schematische Schnittdarstellung der erfindungsgemässen Vorrichtung zur Meniskusrefixation in zusammengebauten Zustand;
Figur 3 zeigt eine schematische Darstellung einer Seitenansicht der Vorrichtung zur Meniskusrefraktion bei einem weiteren Implantationsschritt;
Figur 4 zeigt eine schematische Schnittdarstellung der Seitenansicht der Vorrichtung zur Meniskusrefixation, gemäss Figur 3;
Figur 5 zeigt eine schematische Darstellung einer Seitenansicht der Vorrichtung zur Meniskusrefixation bei einem weiteren Implantationsschritt;
Figur 6 zeigt eine schematische Darstellung einer Seitenansicht eines Meniskus mit einem Riss;
Figur 7 zeigt eine schematische Darstellung einer Draufsicht eines Meniskus nach Figur 6, wobei der Riss mit einer erfindungsgemässen Vorrichtung nach den Figuren 1 bis 5 refixiert wurde.

### AUSFÜHRUNGSBEISPIEL

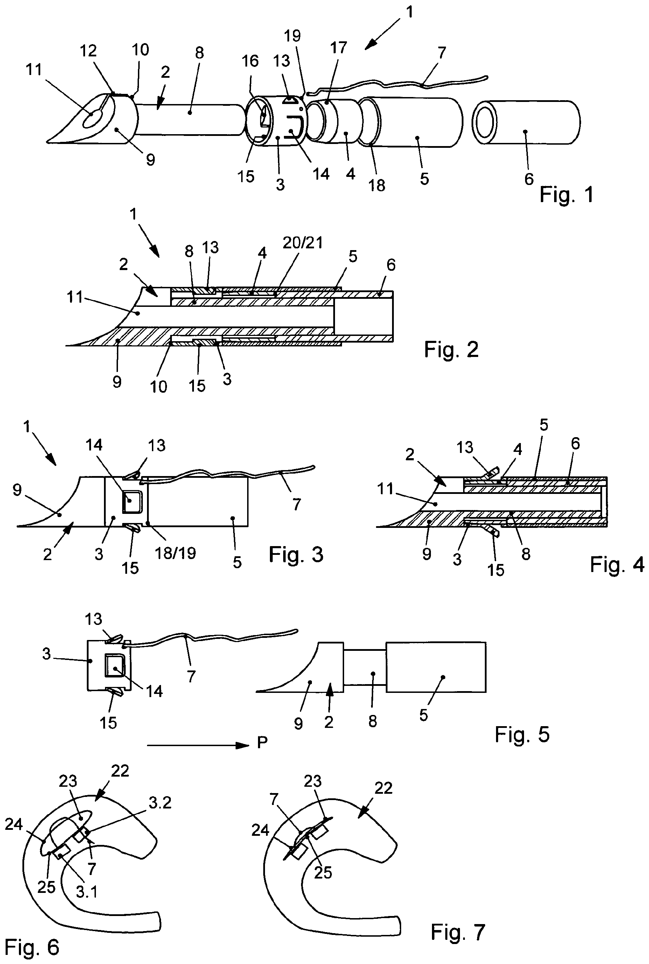

Figur 1 zeigt eine erfindungsgemässe Vorrichtung 1. Die Vorrichtung 1 umfasst eine Nadel 2, ein Implantat 3, einen Spreizring 4, einen Rohrschaft 5, eine Druckstange 6 und einen Faden 7.

Die Nadel 2 umfasst einen Schaft 8 und ein Vorderteil 9. Der Schaft 8 der Nadel 2 weist einen geringeren Durchmesser als ein Vorderteil 9 der Nadel 2 auf. Dadurch bildet die Nadel 2 einen Absatz 10 aus. Nadel 2 weist parallel zu ihrer Längsachse eine Bohrung 11 auf. Des Weiteren umfasst die Nadel 10 in ihrem Vorderteil 9 einen Schlitz 12. Der Schlitz 12 durchdringt das Vorderteil 9 bis zu der Bohrung 11. Analog weist der Schaft 8 der Nadel 2 einen nicht dargestellten Schlitz auf. Der nicht dargestellte Schlitz verläuft von einem Ende der Nadel 2 bis zu dem Vorderteil 9 der Nadel 2.

Das Implantat 3 ist ein Rohrstück, mit einem Innendurchmesser, der größer als ein Aussendurchmesser des Schafts 8 der Nadel 2 ist. Des Weiteren weist das Implantat 3 vier abspreizbare Teilflächen 13, 14, 15 und 16 auf.

Figur 2 zeigt die erfindungsgemässe Vorrichtung 1 in einem Zustand, in dem die Vorrichtung 1 in Gewebe, insbesondere ein Kniegelenk eingebracht wird.

Wie in Figur 2 dargestellt, ist das Implantat 3 auf den Schaft 8 der Nadel 2 aufgeschoben. Der Absatz 10 der Nadel 2 dient als Anschlag für das Implantat 3 und verhindert, dass das Implantat 3 über das Vorderteil 9 der Nadel 2 rutschen kann.

Ebenfalls auf dem Schaft 8 der Nadel 2 positioniert ist der Spreizring 4. Der Spreizring 4 ist ein Rohrstück, das an einer Vorderseite eine Fase 17 aufweist. Ein Aussendruchmesser des Spreizrings 4 ist geringer als ein Innendruchmesser des Implantats 3 ausgebildet. Ein Innendurchmesser der Spreizrings 4 ist grösser als ein Aussendurchmesser des Schafts 8 der Nadel 2 ausgebildet.

Wie ebenfalls aus Figur 2 hervorgeht ist auch der Rohrschaft 5 auf dem Schaft 8 der Nadel 2 positioniert. Eine vordere Kante 18 des Rohrschafts 5 schliesst an eine hintere Kante 19 des Implantats 3 an. Der Spreizring 4 ist in dem Rohrschaft 5 aufgenommen.

Des Weiteren ist auf dem Schaft 8 der Nadel 2, zumindest teilweise das Druckstück 6 aufgenommen. Das Druckstück 6 ist ebenfalls ein Rohrstück, mit einem Aussendruchmesser, der geringer ist als ein Innendurchmesser des Rohrschafts 5. Das Druckstück 6 ist ebenfalls zumindest teilweise in dem Rohrschaft 5 aufgenommen. Mit einer Vorderkante 20 liegt das Druckstück 6 an einer Hinterkante 21 des Spreizrings 4 an.

Die Funktionsweise der vorliegenden Erfindung ist folgende:
Figur 6 offenbart eine schematische Seitenansicht eines Meniskus 22 mit einem Riss 23.

Zur besseren Übersichtlichkeit wird ein erstes Implantat in den Figuren 6 und 7 mit dem Bezugszeichen 3.1 und ein zweite Implantat mit dem Bezugszeichen 3.2 bezeichnet. Bevorzugt sind diese Implantate baugleich und entsprechen dem Aufbau des beschriebenen Implantats 3.

Zur Meniskusrefixation werden zwei Nadeln, die jeweils mit einem Implantat 3.1 bzw. 3.2 und einem Faden 7 und einem Spreizring bestückt und über eine nicht dargestellte gemeinsame Bedieneinrichtung verbunden sind, parallel zu dem Riss 23 in das Gelenk eingebracht.

Zusätzlich zu der gemeinsamen Bedieneinrichtung sind die beiden Nadeln auch über eine nicht dargestellte Fadenbrücke verbunden.

Bevorzugt sind die beiden Nadeln in einer gemeinsamen Hülle eingebracht. Die gemeinsame Hülle dient dem Schutz der Nadeln und der Bestückungsteile, sowie dem einfacheren Einführen der Vorrichtung in das Gelenk.

In bevorzugten Ausführungsbeispielen ist der Rohrschaft so ausgebildet, dass er zwei Nadeln, die jeweils mit einem Implantat, einem Faden, einem Spreizring und evtl. auch einem Druckstück bestückt sind, aufnehmen kann.

Für die weitere Beschreibung der Verwendung der erfindungsgemässen Vorrichtung 1 ist es unerheblich, ob die Nadeln gemeinsam in einer Hülle oder jeweils in einer eigenen Hülle aufgenommen sind.

Die erste Nadel 2, bestückt mit dem ersten Implantat 3.1 wird aus dem Rohrschaft 5 nach vorne geschoben und wie am Verlauf des Fadens in Figur 6 erkennbar parallel zu dem Risses 23 in den Meniskus eingebracht. Im folgenden wird allgemein das Einbringen eines Implantats 3, stellvertretend für die Implantation des Implantats 3.1 beschrieben.

Durch Betätigen des Druckteils 6 wird der Spreizring 4, wie in den Figurern 3 und 4 gezeigt, so unter das Implantat 3 geschoben, dass die Teilflächen 13, 14, 15 und 16 abgespreizt werden. Dadurch ergibt sich der Vorteil, dass das Implantat 3 in dem Knorpel bzw. Meniskusgewebe verankert wird. Durch das Eintreiben des Spreizringes 4 in das Implantat 3 wird gleichzeitig der Faden 7 fest in dem Implantat 3 fixiert. Durch die proximalseitige Abspreizung der Teilflächen 13, 14, 15 und 16 wird sichergestellt, dass ein Herausziehen des Implantats unmöglich wird. Vorteilhafterweise erfolgt das Fixieren des Fadens 7 ohne Knoten, der später an Knorpelgewebe oder am Knochen reiben könnte.

In medizinischer Literatur wird statt des Begriffs Implantat oft der Begriff Anker oder Fadenanker für ein Bauteil das dem Implantat 3 der vorliegenden Erfindung entspricht, verwendet.

Anschliessend wird die Nadel 2 aus dem Implantat 3 entfernt. Dazu wird die Nadel 2, wie in Figur 5 dargestellt, in Pfeilrichtung P rückwärts aus dem Implantat 3 und dem in Figur 5 nicht dargestellten Spreizring 4 gezogen. Dabei dient das Implantat 3 als Widerlager für das Vorderteil 9 der Nadel 2. Das Vorderteil 9 der Nadel 2 ist auch durch den Schlitz 12 und die Bohrung 11 so ausgeformt, dass es beim Erreichen einer bestimmten Kraft so plastisch verformt wird und in sich zusammenfällt, dass es durch das Implantat 3 in Pfeilrichtung P entfernt werden kann.

In bevorzugten Ausführungsbeispielen ist zumindest das Vorderteil 9 der Nadel 2 aus einem Material gebildet, das ausreichend hart und widerstandsfähig genug ist, um in Gewebe, insbesondere Knorpelgewebe einzudringen. Trotzdem ist das Material des Vorderteils 9 der Nadel 2 weicher ausgebildet, als ein Material des Implantats 3, so dass das Implantat 3 als Widerlager dienen kann.

Nach dem Entfernen der ersten Nadel 2 verbleibt das Implantat 3, mit dem Faden 7 und dem Spreizring 4 im Meniskus 22.

Mit der zweiten Nadel, die mit dem bereits eingebrachten Implantat 3.1 über den Faden 7 bzw. die Fadenbrücke verbunden ist, wird das zweite Implantat 3.2 analog zu dem Riss 23 positioniert.

Dabei wird der Faden 7, wie in den Figuren 6 und 7 gezeigt, als Schlaufe mit den Implantaten 3.1 und 3.2 über dem Riss 23 festgelegt. Das Einbringen des zweiten Implantats 3.2 erfolgt analog zum Fixieren des ersten Implantats 3.1.

Bevor der Spreizring 4 in das zweite Implantat 3.2 zum Fixieren des Fadens 7 und zum Abspreizen des Teilflächen 13, 14, 15 und 16 eingetrieben wird, wird durch Zug an dem Faden 7 der Riss 23 geschlossen, wie in Figur 7 gezeigt. Abschliessend wird die zweite Nadel aus dem Implantat 3.2 entfernt und der Faden 7 abgeschnitten.

### Bezugszeichenliste

| | | | | | |
|---|---|---|---|---|---|
| 1 | Vorrichtung | 34 | | 67 | |
| 2 | Nadel | 35 | | 68 | |
| 3 | Implantat | 36 | | 69 | |
| 4 | Spreizring | 37 | | 70 | |
| 5 | Rohrschaft | 38 | | 71 | |
| 6 | Druckstange | 39 | | 72 | |
| 7 | Faden | 40 | | 73 | |
| 8 | Schaft | 41 | | 74 | |
| 9 | Vorderteil | 42 | | 75 | |
| 10 | Absatz | 43 | | 76 | |
| 11 | Bohrung | 44 | | 77 | |
| 12 | Schlitz | 45 | | 78 | |
| 13 | Teilfläche | 46 | | 79 | |
| 14 | Teilfläche | 47 | | | |
| 15 | Teilfläche | 48 | | | |
| 16 | Teilfläche | 49 | | | |
| 17 | Fase | 50 | | | |
| 18 | Kante Rohrschaft | 51 | | | |
| 19 | Kante Implantat | 52 | | P | Pfeilrichtung |
| 20 | Kante Druckstück | 53 | | | |
| 21 | Kante Spreizring | 54 | | | |
| 22 | Meniskus | 55 | | | |
| 23 | Riss | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Vorrichtung (1) zur Meniskusrefixation, mit mindestens
- einer Nadel (2) und mindestens
- einem Implantat (3)
wobei das Implantat (3) mindestens eine abspreizbare Teilfläche (13, 14, 15, 16) aufweist, **dadurch gekennzeichnet, dass** die Vorrichtung einen Spreizring (4), geeignet die Teilfläche (13, 14, 15, 16) des Implantats (3) abzuspreizen, aufweist, dass das Implantat (3) einen Faden (7)
umfasst,
und dass der Spreizring (4) geeignet ist den Faden (7) an dem Implantat (3) zu fixieren.

2. Vorrichtung nach Anspruch 1, **gekennzeichnet durch** eine Druckstange (6), geeignet zum Einbringen des Spreizrings (4) in das Implantat (3).

3. Vorrichtung nach einem der vorhergehenden Ansprüche, **dadurch gekennzeichnet, dass** die Nadel (2) durch einen Kraft plastisch verformbar ist.

4. Vorrichtung nach einem der vorhergehenden Ansprüche, **gekennzeichnet durch** einen Rohrschaft (5), geeignet zur Aufnahme der Nadel (2).

5. Vorrichtung nach einem der vorhergehenden Ansprüche, **gekennzeichnet durch** insbesondere eine flexible Druckstange mittels der eine Kraft entlang einer gebogenen Nadel mittels einer Druckplatte eingebracht werden kann.

## Claims

1. Device (1) for meniscus reattachment, with at least
- one needle (2) and at least
- one implant (3)
wherein the implant (3) has at least one splayable partial area (13, 14, 15, 16), **characterized in that** the device has a spreader ring (4) suitable for splaying the partial area (13, 14, 15, 16) of the implant (3), **in that** the implant (3) comprises a thread (7), and **in that** the spreader ring (4) is suitable for securing the thread (7) on the implant (3).

2. Device according to claim 1, **characterized by** a pressure rod (6) suitable for inserting the spreader ring (4) into the implant (3).

3. Device according to one of the preceding claims, **characterized in that** the needle (2) is able to undergo plastic deformation by force.

4. Device according to one of the preceding claims, **characterized by** a tube shaft (5) suitable for accepting the needle (2).

5. Device according to one of the preceding claims, **characterized by** in particular a flexible pressure rod by means of which a force can be inserted along a bent needle by means of a pressure plate.

## Revendications

1. Dispositif (1) de réattachement du ménisque doté au moins
- d'une aiguille (2) et d'au moins
- un implant (3)
dans lequel l'implant (3) comporte au moins une zone partielle pouvant être évasée (13, 14, 15, 16), **caractérisé en ce que** le dispositif comporte un anneau d'écartement (4) adapter pour évaser la zone partielle (13, 14, 15, 16) de l'implant (3), **en ce que** l'implant (3) comprend un filetage (7), et **en ce que** l'anneau d'écartement (4) est adapté pour fixer le filetage (7) sur l'implant (3).

2. Dispositif selon la revendication 1, **caractérisé en ce qu'**une tige de pression (6) est adaptée pour insérer l'anneau d'écartement (4) dans l'implant (3).

3. Dispositif selon l'une des revendications précédentes, **caractérisé en ce que** l'aiguille (2) peut subir une déformation plastique par la force.

4. Dispositif selon l'une des revendications précédentes, **caractérisé en ce qu'**un arbre en tube (5) est adapté pour accepter l'aiguille (2).

5. Dispositif selon l'une des revendications précédentes, **caractérisé par**, en particulier, une tige de pression souple par le biais de laquelle une force peut être insérée le long d'une aiguille pliée au moyen d'une plaque de pression.
